# EUROPEAN PATENT APPLICATION

(11) **EP 2 767 281 A1**
(43) Date of publication of application: **20.08.2014**
(21) Application number: 11873850.9
(22) Date of filing: 12.10.2011
(51) Int. Cl.: A61K 31/366

(54) **BASE POWDER FROM THE RED RASPBERRY RUBUS IDAEUS AND ACTIVATED MICRONIZED ZEOLITE FOR ATTENUATING NICOTINE ADDICTION, METHOD FOR THE PREPARATION THEREOF AND USE THEREOF**

(71) Applicant: Universidad Autonoma del Estado de Hidalgo, 42000 Pachuca de Soto Hidalgo (MX); Alanis Ortega, Javier Isaias, 62550 Jiutepec, Morelos (MX); Pérez-Pastén Borja, Ricardo, 42086 Pachuca Hidalgo (MX)
(72) Inventor: ALANÍS ORTEGA, Javier Isaías, 62550 Jiutepec Morelos (MX); PÉREZ-PASTÉN BORJA, Ricardo, 42086 Pachuca Hidalgo (MX); FILARDO KERSTUPP, Santiago, 42086 Pachuca Hidalgo (MX); ATITLÁN GIL, Alfonso, 42086 Pachuca Hidalgo (MX)
(74) Representative: Elzaburu S.L.P.
(86) International application number: PCT/MX2011/000124
(87) International publication number: WO 2013/055197

(57) **Abstract**

The present invention relates to a base powder for preparing functional beverages, soft drinks or an additive for food products, which is made with red raspberry from Rubus idaeus and with clinoptilolite zeolite contained in the ZAM® (activated micronized zeolite). The powder is prepared with dehydrated fruit juice. The raspberry juice extract is obtained by slowly heating (without exceeding 70°C) a pot containing the ground fruit or a receptacle with fruit, using the bain-marie method, without exceeding 70°C. The juice is stabilized, dehydrated optionally mixed with sugar substitutes, sweeteners or fiber, and, lastly, homogenized. The object of the present invention is to provide a food-grade base powder of natural origin that is totally different from current presentations of red raspberry on the market. Likewise, the present invention seeks to utilize the therapeutic properties of said fruit as a coadjuvant in controlling active and passive tobacco consumption, nicotine detoxification and nicotine addiction, and also detoxification in the case of other toxic substances in tobacco smoke, as an antioxidant, antimutagenic, anticancer agent and immunostimulant. It is 100% rehydratable in water, easy to transport and has a shelf life in excess of one year.

## Description

### FIELD OF INVENTION

The field of this invention is to protect the intellectual property, ensure the product and the process to obtain the base powder made with raspberry (*Ideaus Rubus*) fruit and activated and micronized zeolite (ZA), used to prepare functional beverages, refreshing beverages or food products, including encapsulated product or in tablet presentation. The product obtained mixing the Ideaus Rubus and the ZAM® (activated and micronized zeolite), has the capability to attenuate the addiction and the withdraw syndrome caused by nicotine.

### BACKGROUND OF THE INVENTION

The growing trend to use natural plants, fruits and minerals to promote health and contribute to the fight against different illness, increase consumers interest for new food products that are being continuously developed by the industry. The abundant content of the ellagic acid in the rubus idaeus, make this as an ideal fruit for propose it, as a antioxidant product, as an antimutagenic product, anticancer product (in some types of cancer), as well as an alternative to the tobacco addiction. Based on the fruit efficacy as antioxidant and antimutagenic, as well as the modification of the nicotine metabolism and in the addictive effects of nicotine, shows the need to develop different ways to obtain the benefits of this fruit. The consumption of this fruit in its natural form and not ripe, using it daily could be repulsive. In other side, the ZAM® zeolita-clinoptilolita micronized activated zeolite of high quality and purity, act as a functional vehicle (carrier) of the fruit and herbal extracts, vitamins, organic and synthetics molecules; used in pharmaceuticals preparations, food supplements and cosmetics. So the application of the zeolite as a food supplement vehicle, transform the ZAM® (activated and micronized Zeolite) as a mechanism that allows to carry ellagic acid, in the raspberry in a major quantity and with better quality, potentiating the effects as antioxidant, anti-mutagenic and anti- carcinogenic. In addition the ZAM (activated and micronized zeolite) also has antioxidant properties, anti-mutagenic and anti-carcinogenic, as well as the detoxify capacity catching nicotine and some other toxic substances in tobacco smoke.

The benefic effect of the fruits and minerals, as well as the growing interest of the consumers for their health condition, promotes that the industry continuously offer new food products to respond to the population demands. The concept of the functional foods, marks the beginning of the development of food products to improve the health and reduce the risk of becoming sick. A functional food is that one that has components that provides benefits for health, beyond its basic nutrition Its about a food product or beverage that produce physiologic benefits, in function of the content of one or many natural active ingredients, aggregated or modified, changed by technology or biotechnology.

The need to have food products that have more benefits for health also is supported by the socioeconomic changes and demographics, happening in the population; reason why professionals from health and food industry are looking for different forms to control these changes in a more efficient way. As an example of this, the phytochemicals use, so called too as phytodrugs have taken relevance in the therapeutic arsenal use globally.

Nowadays, the nutrition is changing rapidly, with focus in certain interest areas. The nutritional deficiencies aren't anymore the main priorities of the research; nowadays the interest is in the relation between the food and the non transmissible chronic diseases, and the effects of the nutrition over the cognitive, immune functions, the work ability and athletic performance. In the other side, the consumers are increasingly aware of self care and are searching in the market those products that contribute to health and wellbeing. These foods, that promote health, generically have been called functional foods and the enterprises that produce them, are showing a fast global expansion.

The functional foods have the particular characteristic that some of their components affect in a specific and positively way, over some of the organism functions, promoting a physiologic or psychological effect beyond of their nutritive traditional value. The additional effect can be their contribution to keep the health and wellbeing, or the decrease in the risk of getting sick.

Another example is the use of phytochemicals, that have taken relevance into the therapeutic arsenal worldwide. The phytopharmacology and the phytotherapy, are recognized and used by the traditional medicine. In the plants or in their parts, as well as in the fruits, that are used to generate the functional foods, the active ingredients are always in biologic balance by the presence of complementary substances, that are potentiated between each other, so they are not accumulated in the organism; and fundamentally because their not desired effects are limited, and also they employ low investment technologies and raw materials with the consequence reduction in cost. (Hernández, 2003).

In particular, alcohol and tobacco are the most abused commonly used substances The extract of Hypericum perforatum L, is an example of pytotherapy compound, has being used for the treatment of low to moderated depression, caused by the abuse of the mentioned substances. These studies were focused on the alcohol and nicotine dependence (Tayfun 2001).

The smoke of the tobacco is a complex mix of chemicals agents which besides or being toxic, mutagenic and carcinogenic, contains nicotine, which is the responsible of the smoking addiction, is the component that the smokers are looking for.

Once the nicotine enters in the circulatory system, the active nicotine is eliminated from the organism as cotinina. The responsible enzymes for the catalysis in this metabolic reaction are the cytochrome P450 2A6 (in humans) and together with an aldehyde oxidase, conform the limiting stage of the nicotine metabolism. The persons that present deficiencies in the function of this reaction, because of the presence of inactive alleles of the enzyme CYP2A6, present a diminished capacity to metabolize the nicotine; it has being noticed that these persons have less susceptible to smoke. Additionally to the nicotine, the CYP2A6 in humans, metabolize other xenobiotic compounds, such as tobacco nitrosamines and other toxic components. Recent experimental evidences show that the inhibition of the CYP2A5 enzyme in mice, similar to CYP2A6 in human, reduces dramatically the tumoragenesis originated by the nitrosamine.

Until now, the studies that suggest the influence of the nicotine metabolism and its relationship with the development and maintenance of the addiction are extense; meanwhile there are few experimental evidences that relate the changes in the nicotine metabolism and its effect in addictive conduct; and the studies that analyze nicotine metabolic inhibition, are mainly focused on the toxic effects of this alkaloid. Denton et.al.(2004) demonstrated that the analog β - (nicotirine), product of the nicotine combustion, inhibits the CYP2A6 enzyme, in the same way that the quinidine at high dosages, is also able to inhibit this enzyme (Hirano and Mizutani, 2003). Rahnasto et.al. (2005) developed a β - (naftalene) analog series, all capable to inhibit specifically the CYP2A6. No one of the mentioned cases, have considered the relationship of this property with the addictive conduct. In other side, some studies have demonstrated that some polyphenolic agents are capable to protect from mutagenesis and carcinogenesis induced by nicotine, thanks to their capacity to inhibit their metabolism. Miller et.al. (1993), demonstrated how the polyphenolic compounds of the natfoflavones family, can reduce the mutagenesis inducted by nitrosamines in s.tyhpimurim, this effect is explained by the inhibition of the set of the enzymes of the citochrome P450. Castonguay et.al. (1993) proved that the polyphenolic compounds coming from of the diet, are able to reduce the tumorogenesis in mice that were exposed to nitrosamines; meanwhile Zhang (1993) have related the protective effect whith the enzyme CYP2A6 inhibition. In this sense, the ellagic acid (EA) (2,3,7, 8 - tetrahydrobenzopyran [5, 4, 3 - cde] benzopyran -5-10 dione) is a polyphenolic compound that is located in fruits like raspberry, cranberry, pomegranate, similar fruits, in the walnut.inwood; has been demonstrated that possesses different pharmacologic effects like anticarcinogenic, antibacterial and anti-inflammatory. In in-vitro studies it has being observed that affects the expression of genes p53 y 021 and produces detention in phase G1 and apoptosis in cancer cells. Also, inhibits the growth of cancer cells modifying the activity of the topoisomerase I and II, girase and polymerase enzymes. Also can act as antioxidant against large chain peroxil radicals, and prevent the propagation of the mediated reactions of free radicals.

Intra peritoneal injection of EA is not effective against lung tumorogenesis benzo [a] pyrene. In contrast, it have been observed a 90% inhibition of lung tumorogenesis benzo [ a] pyrene in mice feed by EA at a dose of 400 mg/kg of EA. Boukharta (2002) showed that the EA is able to reduce the tumorigenicity of the NNK nitrosamine in a concentration dependent manner . At a dose of 4g/kg inhibited tumor multiplicity by 54 %. Multiple observations have led to different hypotheses of the mechanisms preventing tumorogenesis of EA, the main one is based in the ability to inhibit the activation of various carcinogens mediated by CYP2A6, the phase II enzymes induction involved in detoxification of the body. In addition it has been shown that the EA is a modifier of the nicotine-induced genotoxicity in lymphocytes T (Stoner, 2001; Lei 2001; Falsaperla, 2005; Adluri, 2007).

The EA, by its induction of various detoxification enzyme systems is a potential inhibitor of nicotine addiction and withdrawal, as well as a detoxification agent of many tobacco smoke toxic substances. On the other hand the EA is a molecule capable to precipitate proteins and alkaloids (Public Health and Nutrition, 2006); the nicotine is an alkaloid, which when it is precipitated it would be limited to get to their metabolic cycle, with consequent limitation of the addictive process.

In summary, ellagic acid, is an example of one type of polyphenolic compound that acts as a phytochemical, has a variety of biological activity, antioxidant, antiinflammatory, and antifibrosis effect; induces different detoxification enzyme systems, and inhibits reactions by blocking nitrates. Also blocks various carcinogens, reducing benzopyrenes bioavailability in the gastrointestinal tract. It also acts as a modifier of the nicotine-induced genotoxicity, a positive modulation of the damage it causes to T cells. In other studies it was found that the raspberries, in their natural state, can lead to significant increases in removing nicotine, favoring the process of smoking cessation (Milano, 2001).

Likewise, it has been identified that the zeolites, as volcanic minerals originaly, have a powerful detoxifying action, being the only minerals known negatively charged on earth that, naturally, attract and adsorbed positively charged toxins.

By its composition, the zeolite - clinoptilolite is a structured silico - aluminate tetrahedral crystal, often stacked in pairs double or quadruple. The Zeolite-clinoptilolite has a rich network of micropores and microchannels filled with semi-exchangeable cations of calcium, sodium, magnesium and potassium, which may be exchanged with nitrogen and oxygen free radicals.

Several studies support the anti-cancer, antioxidant and detoxifying activity of the zeolite. Kresimir (2000) showed the zeolite - clinoptilolite as an adjunct in anticancer therapy and in 2002 (Kresimir, 2002) demonstrated the immunostimulatory effect of natural cliniptilolita as a possible mechanism of its antimetastatic activity; Kresimir et.al. ( 2004 ) showed an antioxidant and inmunostimulating effect of the natural zeolite, while Grace and Kresimir (2004) demonstrated the antiviral properties to the clinoptilolite. Tomasevic (2005) demonstrated the medical application for removing lead with natural zeolite-clinoptilolite.

To reduce the toxicity of tobacco, several combustion systems have being used (Baker, 2006 and Ding et al. 2007). In these systems, a catalyst has been added, which is mixed with the tobacco or used as filter in the tip cigarettes. Most of these systems involve different materials zeolite or alumino-silicate. Early attempts (Seeofer et al., 1980), a compound was used with the formula M2M'Ru06, where M was a divalent metal, M' is a trivalent rare earth metal, Ru having a valence of 5, and M and M' are capable of forming a network with Ru ions; this compound, when mixed with tobacco or is incorporated in the cigarette paper or in the filter helps reduce the NO and CO of the tobacco smoke. Rongved ( 1997) described a compound to produce less toxic substances as carbon monoxide in the flue gas, by adding inert, stable and non-contaminating solid catalysts in a mixture with the tobacco or as a filter, using a metal catalyst, as vanadium pentoxide, molybdenum trioxide or rhodium oxide. More recently , Li et al. (2003), disclosed the use of nanoparticles of Fe2O3, CuO, TiO2, CeO2, Ce2O3, Al2O3, Y2O3 doped with Zr, Mn2O3 combined with Pb, to increase the conversion of CO to CO2. On the other hand, it has been described that the organic salts of potassium can be used as additives to tobacco and in the burning cigarette, may reduce the yields of CO and nicotine, (Li et al., 2003). In 2004, Li et al, describe the use of an oxidizing catalyst, based on iron oxide nanoparticles, which is generated in situ when the cigarette is burning, and is able to increase the conversion of CO and NO to CO2 and N2, respectively.

The use of zeolites and other additives aluminum- silicates has been described by several authors. Cvetkovic et al., (2002) described the use of a catalyst based on a combination of Cu-zeolite and ZSM-5 to reduce the amount of NO and NOx in the tobacco conventional smoke. This catalyst can be added to the cigarette filter or can be directly mixed with tobacco. This proposal is based on the adsorption or diffusivity properties of the material. Meier et al., (2001 and 1999 ) also describe the use of zeolitic materials as additives, and Xu and Zhu (1985); its mesoporous material (MCM-48 , NaA Zeolite NaY KA and NaZSM-5, SBA-15 y MCM-48) used as additives to tobacco to cigarettes, and can reduce the content of nitrosamines, by selective adsorption, into the smoke snuff sucked by active smokers directly cigarette. Gao et al., (2009) also describe the use of CAS -1 in the cigarette filter for selectively absorbing nitrosamines. Yong et al., (2006) proposed the use of MCM- 48 and Ce-MCM48 to reduce the content of polycyclic aromatic compounds in smoke tobacco main stream. Chen et al., (2006) presented NaY zeolite carbon, which activates and oxidized carbon nanotubes in the filter cigarette and was found an important reduction of tar and nicotine. Also reported by Branton et al.,(2009) the important reductions in fumes composition of activated carbon when the zeolitic compounds are introduced into the filter tip.

Other studies have reported the effect of three commercial zeolites (Husy, Hb, HZSM-5) and a synthesis of catalysts, Al-Si-MCM41 (crystalline rolling material, silicate obtained by a templating mechanism ) in the composition of the mainstream smoke obtained from one brand of cigarettes of tobacco Virginia has been studied. Catalysts were directly mixed with tobacco. The results suggest that the inclusion of Catalyst Al-Si-MCM41 directly with the tobacco in cigarettes could be an effective method for reducing the presence of many toxic and carcinogenic compounds in the mainstream smoke.

Therefore, the ZAM ® (activated and micronized zeolite potentiates the effects of ellagic acid and in general the raspberry powder effect, acting as a vehicle increasing the bioactivity and bioavailability of raspberry powder and its components. The patent applicants have shown that the ability of pure ellagic acid and reconstituted juice raspberry based powder and also the same powder used as a food additive in other products, the first motive of the present patent, are able to modify the addictive conduct and the nicotine withdraw syndrome in dosage-dependant mice. In the other side, the patent applicants, also have demonstrated that ZAM® (activated and micronized zeolite), manufactured by Granding International SA de CV , from natural zeolite - clinoptilolite, having an average particle size of 3 microns, a surface area of 24 m2/g, has been thermally activated, so its bioactivity and bioavailability are increased, allowing it to act as a carrier functional for raspberry powder. Also, besides being a potent antioxidant, anticarcinogenic and being able to trap nicotine and other toxic substances from tobacco smoke, is able to absorb toxins such as NH3, hydrocarbons, C1-C4, CO₂, H₂S, SO₂, NOx, aldehydes , besides heavy metals like lead, cadmium, cesium mercury, etc. is the second motive of applying this patent. To verify product novelty, a search was performed on different databases to determine the state of the art for the product under this application. The databases consulted were: National Bank of Patent (BANAPANET), United States Patent and Trademark Office (USPTO) and the European Patent and Trademark Office (EPO) in the combination of words, technical terms or synonyms and / or codes classification also as: DUST AND RED RASPBERRY AND FUNCTIONAL DRINK, ZEOLITE and CLINOPTILOLITE and MICRONIZED. From this search more than 200 documents were obtained, but only those that seemed to be related to the subject matter of this document were selected and are listed below:
**a.** Patent Application: KR20040010390, Date: 31-01-2004; Antiinfluenza Virus agent and composition containing the same and food or drink; Kazafumi, S. **et al.**
   In this application a standardized formula for the following fruits and plants is obtained: Rubus idaeus, Fragara vesca, Rubus fruticosus, Ficus cariaca, Chenopodium album, Agrimonia eupatoria, Eucalyptus globulus, Prunus persica, Malus pumila, Viola odorata, Lindera umbellata, Paullinia cupana, Buddelia officinalis, Commelina communis, Capsella bursapastoris, Rabdosia japonica. Which is obtained by dehydration juice and hydroalcoholic extracts at temperatures of 4 to 5 ° C, prioritizing stability thermolabile phytochemical agents. The biological activity attributed to him is that of an anti - influenza (flu) agent, attributed to immunostimulatory properties.
**b.** Patent Application U.S. 2004/0013749 A1, Date: 22-01-2004; Antioxidant and **inmune boosting composition and methods of using; Young, D.G.et al.**
   In this application a standardized formula raspberry (Rubus idaeus) is obtained. The formula is obtained by dehydration of the juice temperature of 4 to 5 ° C and pH below 5, prioritizing stability thermolabile phytochemical agents. The biological activity attributed to them is that of an antioxidant and immunostimulant agent. The standardization of the formula is made based on the antioxidant capacity of said extract.
**c.** Patent Application**:** PA/a/2005/005479; PCT/EP2003/012975; Date: 23-01-2005**, Nutraceutical compositions which comprise epigallocatechin gallate and raspberry ketone; Weber, P. et al.**
   In this application claims a standardized formula gallate epigalato and ketone obtained from raspberry (Rubus idaeus). The formula is obtained by dehydration of the hydroalcoholic extract fractionated at temperatures of 4 to 5 ° C and a pH below 5 and extraction methods , prioritizing stability thermolabile phytochemical agents. The biological activity is attributed is preventive against cancer and neoplasm. The standardization of the formula is made based on the concentration of gallate - epigalato.
**d.** Patent: PA/a/1997/002984; Date: 04/24/1997: Removal of iron and manganese by adsorption-oxidation on natural zeolite, method of forming the contact medium and regenerate its adsorption capacity.
   In this patent technique and methodology is registered in which the physical and chemical characteristics of the clinoptilolite type zeolite, is used for removal or removal of iron and manganese metal ions that are found in the water of some underground sources of supply.

The product for which a this patent is required has different obtaining methods as the aforementioned documents, and also have different applications; is a base powder raspberry with zeolite to produce a functional beverage, as an additive to other foods or other products, that is obtained from a standard fruit juice extracted by the method of pot or bath at temperatures above 50 to 70 ° C. This juice is then dehydrated by a spray dryer at a temperature of 110 °C. This procedure favors the thermo-stable components especially ellagic acid. Also ellagic acid is the component to which ot is attributed the biological activity, which aids in the treatment of nicotine addiction, the body immunostimulation, antioxidant, antitumor and anticancer activity. which is enhanced by adding the ZAM ® (activated and micronized zeolite), for its activity as a carrier, as a detoxifying , immunostimulant, antioxidant and anticancer.

Therefore the above documents do not represent duplicity, consequence or immediate deduction, or variations of patent applications recovered from the homologous search patents, and consequently the product for which registration patent is requested, meets Innovation condition. In the previous documents was found that in obtaining raspberry products, an extremely low temperatures were used and drying at low temperature and no heating or drying at high temperatures, as it is being in the product object by this patent; further that none of the cited cases, privileges the presence of ellagic acid or the biological effect attributable to it, as an aid in the detoxification of nicotine and therefore in the treatment of anxiety during the quitting smoking process and in the treatment of addiction to nicotine; even more nor reported to the raspberry combined with clinoptilolite zeolite, much less the carrier activity of ZAM ® (activated and micronized zeolite for the components of raspberry powder; and also none mention the improved bioavailability and participation as detoxifying nicotine and other toxic substances in tobacco smoke.

### DETAILED DESCRIPTION OF THE INVENTION

The Product object of the present invention is obtained from the combination of the ZAM ® (activated and micronized zeolite) and raspberry powder according to the process registered in the Mexican Institute of Industrial Property , in patent application number Mx/a/2010/000827 dated 21/ENE/2010 , folio MX/E/2010/004489 ) . So some modifications that improve product under such protection registration are specified. The base powder is used to prepare functional beverages, soft drinks, or also used as an additive for food products and other products for tobacco addiction control treatment. The base powder is made from pulp and dried strawberry raspberry juice fruit and ZAM ® (micronized and activated zeolite that serves as a carrier or vehicle to improve bioavailability of ellagic acid, together with other ellagitannins in the raspberry, as well as detoxifying agent for nicotine and other toxic substances present in tobacco smoke.

The fruit is selected by assessing the clear red color, making sure that it is free of any toxic substances, in accordance with the tolerance regulation allowed by the Secretariat of Health and Welfare for processing fruits.

The raspberry juice extract is obtained using the method of kettle or water bath method, or some other alternative method. Once raspberry juice extract is obtained, a drying process is done to obtain the powder, for example the drying could be done by using a Spray -dryer, or a lyophilization process, or any other alternative method As an option, the powder obtained form raspberry juice extract can be added with any allowed sweetener, for example sugar or aspartame. Also an allowed anti-humectant is added, such as silicon dioxide or tricalcium phosphate. The ingredients are mixed and homogenized.

Once the sweetener and anti-humectant is added, to the powder obtained from extract raspberry juice, ZAM ® (activated and micronized zeolite) is added. The ingredients are mixed and homogenized to obtain the final base powder.

Optionally, the homogenized product may be packaged in glass jar with twist off cap and plastisol seal, or in polyethylene-aluminum laminated pouches and glassed paper, or any other allowed packaging method.

Additionally , the powder reconstituted with water, preserve its minerals content such as calcium, iron, aluminum, magnesium, potassium, silicon, sodium, manganese, sulfates and phosphates , carbohydrates and nitrogenous compounds; as well as vitamins A, B1, B2 and C and chemical components of the ZAM ® (activated and micronized zeolite) such as: SiO₂, CaO, K₂O, MgO, Na₂O.

Also, the powder product has a shelf life of more than one year, keeping its original properties.

The base powder is rehydrated with water instantly and can be used to prepare functional beverages, refreshing drinks or as an additive in food preparations or other products; the product retains all therapeutic properties such as: auxiliary in tobacco addition control, based in the content of ellagitannins, specifically the ellagic acid and the activated and micronized zeolite); promote the precipitation and elimination process of nicotine and helps to maintain low levels of anxiety caused by withdrawal syndrome after stop tobacco smoking, and with this, to lower the administration of nicotine, as well as to diminish the nicotine addiction. These make the product an excellent support therapy to quit tobacco smoking, and because the antioxidant, antimutagenic, anticancer, and inmunostimulatin properties, helps to fight some of diseases caused by tobacco smoking and helps the patient to keep himself free of smoking.

The base powder is rehydrated with water instantly and can be used to prepare functional beverages, refreshing drinks or as an additive in food preparations or other products, retains all its minerals like calcium, iron, aluminum, magnesium, potassium, silica, sodium, manganese, sulfates and phosphates, carbohydrates and nitrogenous composite, plus vitamins A, B1, B2 and C, as well as the chemical components of the ZAM ® (activated and micronized zeolite), SiO₂, CaO, K₂O, MgO, Na₂O.

The base powder is rehydrated with water instantly and can be used to prepare functional beverages , refreshing drinks or as an additive in food preparations or other products, and retains its bioactivity and increased bioavailability, activity provided by the ZAM ® (activated and micronized zeolite), giving it the quality of being an excellent antioxidant to fight free radicals.

The base powder is rehydrated with water instantly and can be used to prepare functional beverages, refreshing drinks or as an additive in food preparations or other products, and provides a bittersweet sui generis solution.

The details and characteristics of the process to develop this base powder from raspberry and ZAM ® (activated and micronized zeolite), to obtain a functional beverage, refreshing drinks or as an additive in food preparations and other products, are shown in detail in the following description:

### Process to obtain of base powder to prepare functional beverages, refreshing drinks or as an additive in food preparations or other products made with red raspberry, Rubus idaeus and ZAM® (activated and micronized zeolite)

**Stage 1.- Main raw material**
   Red raspberry fruits and ZAM ® (micronized and activated zeolite)
Stage 2.- Selection, cleaning and weighting the fruit.
   Raspberry fruit is selected so that it is not too ripe, evaluating its clear red color. Bunches of stems are separated and free of leaves, the peduncles are removed; these are usually divided into two or three pedicels, and the fruits are placed in a clean surface, as for example a clean tray. Raspberry bunches are weighed before dehydrating.
**Stage 3.- Juice extract obtaining and standardization and powder juice extract production.**
   Raspberry juice extract is obtained by using any of the known methods for example:
   1) The method of Marmite.- The fruit previously crushed is placed in a marmite, which is slowly heated but not exceeding 70 ° C, so that the fruit juice begins to flow, during one hour for every 3 kg approximately. This method prevents that the fruit experience overcooking that destroys both the color and the fresh taste, and promotes a higher concentration of ellagic acid in the powder. Once the fruit has released plenty of juice, it is crushed again. Once fruit juice extracted it is filtered by passing through medium pore filter paper.
   2) The water bath method. - The fresh fruit is crushed with a mortar or other means in a large bowl. The fruit bowl is placed in a water bath without exceeding a temperature of 70 ° C to 80 ° C , until the fruit juice begins to flow. Optionally, once extracted fruit juice is obtained it could be filtered using filter paper medium pore.

   Once the juice extracted, to obtain the powder, the extract is subjected to a drying process, for example by spraying using a Spray-dryer equipment type, with an inlet temperature of 110 ° C and outlet of 80 ° C , withan air flow of 600 ml/min and 30 millibars vacuum. It can be done also by lyophilization or any other known method.
**Stage 4.- Using anti-humectants.**
   To the formulation obtained in Stage 3, is added with any allowed antihumectant, for example, silicon dioxide or tricalcium phosphate. Additionally any allowed sweetener may be added, for example, sugar or aspartame.
**Stage 5.- Use of ZAM ® (activated and micronized zeolite) and homogenization in the formulation of the base powder.**
   To the formulation obtained in Stage 4, ZAM ® (activated and micronized zeolite) is added For 250 g of powder raspberry 20 g of zeolite ZAM ® are added. This is mixed and homogenized to obtain the final product.
Stage 6.- Packaging base powder formulation.
   Optionally, the homogenized product may be packaged in glass jar with twist-off cap and plastisol seal, or in polyethylene-aluminum laminated pouches and glassed paper, or by any other allowed method. The product retains its properties for more than 1 year, as the study of shelf life of the product was developed, including extreme temperature and humidity conditions.

### PRACTICAL EXAMPLE FOR CARRYING OUT THE INVENTION

The present example is illustrative and not limiting, such as one skilled in the art will understand, there are variants that fall within the protection of the present invention.

### Process to obtain a base powder to prepare functional beverages, refreshing drinks or to be used as an additive for food preparations or other products, made with red raspberry (Rubus idaeus) and ZAM ® (activated and micronized zeolite).

1) Raspberry fruit with adequate ripeness condition and sanitation is selected.
2) Fruit leaves and stems are removed.
3) Raspberry juice is extracted by placing fresh fruit previously crushed in a kettle. The kettle is heated slowly without exceeding 70 ° C until it starts flowing fruit juice, during about 20 minutes per kilogram of fruit. This method for extracting juice, avoid fruit overcooking and any destruction of both the color and the fresh taste; also this process keep the medicinal properties while allows a higher concentration of ellagic acid. When the fruit has released plenty of juice, it is crushed again.
4) Optionally, the fruit juice is filtered by passing through medium pore filter paper.
5) After extraction of the juice, it is subjected to spray drying process using a spray - dryer equipment, with an inlet temperature of 110 ° C and outlet temperature of 80 ° C, and an air flow of 600 ml/min and vacuum of 30millibars.
6) To prepare the base powder, the spray dried raspberry juice powder is used, and can be prepared for example, in some of the following presentations, mixing the following proportions:
   a) Base powder unsweetened light.- The product consists of 100% powder, obtained from raspberry juice extract spray drying.
   b) Powder base with sweetener.- Were mixed in the following proportions, spray dried extract raspberry juice 50 % and 50 % aspartame.
7) In each of the presentations described above, food grade anti-humectant is added to them, as silicon dioxide or tricalcium phosphate, among others.
8) The ingredients of any of the formulations described above and the anti-humectant are mixed with ZAM ® (activated and micronized zeolite) and homogenized to obtain final base powder.
9) The homogenized final base powder, is packaged in glass jar with twist-off cap and plastisol seal or in polyethylene-aluminum pouch envelopes and glassed paper.

### Main uses of base powder to prepare functional beverages, refreshing drinks or as an additive to food preparations or other products made with red raspberry (Rubus idaeus) and ZAM ® (activated and micronized zeolite).

The powder product corresponds to a natural functional food, nutritional order, which can be used to prepare functional beverages, refreshing beverages as an additive in food preparations and in the preparation of other products.
1. The product is rehydrated with water instantly providing a sui generis solution bittersweet flavor.
2. The product may have various presentations, such as: a) base powder unsweetened light and b ) base powder with sweetener or sweetener, among others.
3. In the case of the base powder presentation with sweetener or sweetener, sugar can be used or fructose or aspartame.
4. The base powder used to prepare functional beverages, refreshing drinks, as an additive in food preparations or other products, uses ZAM ® (activated and micronized zeolite) as a vehicle or carrier when it is ingested; also has the function of detoxifying, to carry, selectively nicotine and other toxic substances present in the tobacco smoke, so they can be eliminated..
5. The base powder can be used to prepare functional beverages, refreshing drinks, and also can be used as an additive in food preparations or other products. The product can be reconstituted with water, fruit juices, any kind of milk, among other ingestible substances, except alcohol, and the product preserves all the fruit properties: color and flavor, as well as the therapeutic properties of the raspberry together with the ZAM ® (activated and micronized zeolite).
6. The base powder reconstituted with water retains its therapeutic properties, such as: antioxidant, anti-mutagenic, anti-carcinogenic and inmunostimulant. The power retains also its detoxification characteristic for nicotine and other toxic substances present in the tobacco smoke and in removing heavy metals in various forms. This characteristic is based in the ellagic acid and zeolite - clinoptilolite content. Also the power is used as an auxiliary in the withdrawal syndrome control associated with nicotine addiction; is able to control anxiety and therefore allows controlling nicotine. Therefore the intake of the power is recommended not only for people with nicotine addiction, but also for non-smokers people exposed to tobacco smoke, based in its function as an auxiliary in the control of anxiety and in the detoxification of nicotine and other toxic substances property, that are present in the tobacco smoke. The mentioned properties in raspberry powder, ellagic acid and ZAM ® (activated and micronized zeolite have been demonstrated by the patent applicants in different biological models; both nicotine withdrawal induced syndrome, and addictive behavior was proved in animals, together with the anxiolytic effect. Likewise it has been demonstrated that the product is auxiliary in the withdrawal syndrome treatment, specifically helping with the anxiety, and also helps to control the addictive behavior in humans.
7. Two experiments in mice were developed using raspberry powder and pure ellagic acid (Sigma Aldrich). One experiment was to assess the effect in the anxiety caused by nicotine withdraw syndrome; and the other experiment was performed to assess the nicotine addiction. Based on the process mentioned in the Mexican Institute of Industrial Property in patent application number Mx/a/2010/000827 record dated 21/ENE/2010, folio MX/E/2010/004489. In the first case the high-cross test was used and in the second test site selectivity paradigm. Nicotine was administered to mice for 14 consecutive days and when it was suspended, anxiety was developed. It was shown that ellagic acid (EA) and raspberry powder (RP) significantly reduce symptoms related to anxiety caused by the refraining from exposure to nicotine, also raspberry powder reduces the natural anxiety that was generated in the group control mice, which means that an anxiolytic effect was obtained even without withdrawal syndrome of nicotine. Furthermore, it was demonstrated that nicotine is able to condition the behavior of mice, in order to receive more stimulation from nicotine; meanwhile the administration of EA and RP are able to modulate this behavior significantly.
8. It was shown that the product is an auxiliary in the withdrawal syndrome control, particularly in relation to anxiety; and in controlling nicotine addiction in humans, using an experiment in which 8 persons used different treatments with obtaining different results as follows:
9. **Treatment 1: -** Two persons, a man and a woman of 54 and 38 years respectively, used ZAM ® (activated and micronized zeolite) at doses of 5 g daily for 14 days.
10. **Treatment 2: -** Two persons, a man and a woman of 45 and 47 years respectively, used the raspberry powder recorded in January 2010 in the Mexican Institute of Industrial Property (patent application number record Mx/a/2010/000827, at , folio MX/E/2010/004489). At a dose of 5 g per day for 14 days.
11. **Treatment 3:** A person, a man of 63 years used a commercial product of raspberry, with similar characteristics to the raspberry powder, mentioned in the previous section, in combination with ZAM ® (activated and micronized zeolite a dose of 5 g per day.
12. **Treatment 4:** Two persons, a woman of 57 and a man aged 53, used raspberry powder registered in the patent application number Mx/a/2010/000827, folio MX/E/2010/004489) in combination with ZAM ®) micronized and activated zeolite) in a dose of 7 g per day for 14 days.
13. **Treatment 5.-** A person, a man of 39 years old, used varenicline, commercial brand, at dose of 1 mg twice daily for 7 days, then 0.5 mg daily for the next 3 days; then 0.5 mg twice daily for the next 4 days, and finally 1 mg during 1 day, every 12 h. This cycle was repeated 12 weeks.
14. Anxiety was assessed by the Hamilton Anxiety Scale (Hamilton , 1969; Bulbena, 2000; APA, 2000 ) and the degree of nicotine dependence with Fagerström test (1991).
15. The Hamilton Anxiety Scale (Table 1) is one of the most used instruments in pharmacological studies of anxiety. It was used according to the criteria established by the American Psychological Association (APA, 2000) , to assess the severity of anxiety in a global manner in patients who met criteria for anxiety or depression and to monitor response to treatment.

**Table 1**

| Establish the intensity which was has accomplished or not during the last month, the symptoms that are describing in forward follow the criteria next. | | | | | | |
|---|---|---|---|---|---|---|
| 0. Absent | | | | | | |
| 1.- Intensity low | | | | | | |
| 2.- Intensity medium | | | | | | |
| 3.- Intensity serious | | | | | | |
| 4.- Incapacity total | | | | | | |
| 1 | Anxious state: Worries, fears the worst happens, anticipated fear, irritability | 0 | 1 | 2 | 3 | 4 |
| 2 | Stress: Feelings of stress, fatigability, startle responding, easy crying, tremors, feeling restless, inhability to relax | 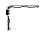 | 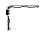 | 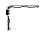 | 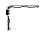 | 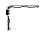 |
| 3 | Fears: To the dark, to the unknown, to be left alone, to the animals, to the traffic, to the crowds. | 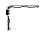 | 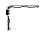 | 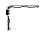 | 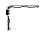 | 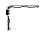 |
| 4 | Insomnia: Difficulty falling asleep, interrupted sleep, unsatisfying sleep and feeling of fatigue on waking, nightmares, night terrors | 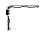 | 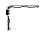 | 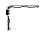 | 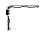 | 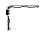 |
| 5 | Intellectual functions (cognitive): Difficulty in concentration, poor memory or poor. | 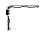 | 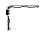 | 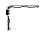 | 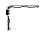 | 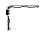 |
| 6 | Depressed mood: Interest lost, to pleasure, to depression, waking up earlier than expected, mood swings throughout the day. | 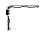 | 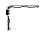 | 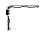 | 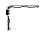 | 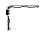 |
| 7 | Muscle somatic symptoms: muscle aches, spasms, cramps, muscle stiffness, twitching, teeth grinding, hesitant voice, increased muscle tone | 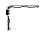 | 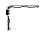 | 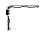 | 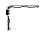 | 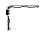 |
| 8 | Somatic sensory symptoms: Ringing in the ears, blurred vision, cold and heat waves, feeling of weakness. Paresthetic sensations (pricks, itching or tingling). | 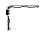 | 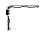 | 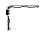 | 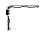 | 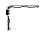 |
| 9 | Cardiovascular Symptoms: tachycardia, palpitations, chest pain (chest), pronounced vascular pulsations, feeling "low pressure" or fainting, arrhythmia | 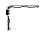 | 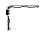 | 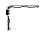 | 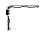 | 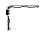 |
| 10 | Respiratory symptoms: Oppression or constriction in the thorax (chest), feeling of choking, sighs, dyspnea (feeling of shortness of breath or respiratory distress). | 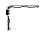 | 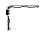 | 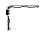 | 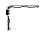 | 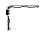 |
| 11 | Gastrointestinal symptoms: difficulties swallowing, flatulence, abdominal pain, burning sensation, heaviness, nausea, vomiting, loose stools, weight loss, constipation. | 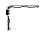 | 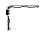 | 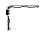 | 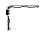 | 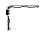 |
| 12 | Genitourinary symptoms: Frequent urination, urgent urination, lack of mesntrual period, menorrhagia, frigidity, premature ejaculation, loss of sex drive, sexual impotence. | 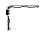 | 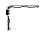 | 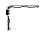 | 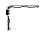 | 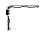 |
| 13 | Symptoms of autonomic nervous system: Dry mouth, fits of flushing, pallor, tendency to sweating, dizziness, headache stress, goosebumps | 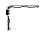 | 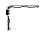 | 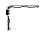 | 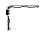 | 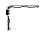 |
| 14 | Conduct during the test: restlessness, impatience, restlessness, trembling hands, puckering frown, worried face, sighing or rapid breathing, burping, tics. | 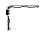 | 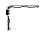 | 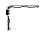 | 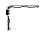 | 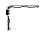 |

16. 14 scale items were evaluated, 13 related to anxiety symptoms and signs, and the last item values the patient's behavior during the assessment interview. In all cases it was applied by a medical doctor before and after each treatment (treatments 1 to 5, described in points 9 to 13 of this section). The interview lasted 25 to 30 minutes before and after treatment. Each item presents a number of signs and symptoms that are helpful in their assessment, although no specific anchor points. In each case both the item intensity and the item frequency were considered. Each item is rated on a scale of 0 to 4 points, since just some questions refer to signs that can only be observed during the interview, each patient was questioned about their situation in the last 2 weeks before the interview (both before starting treatment and once it was completed). This happen for treatments 1 to 4, according to what reported by Bech (1993) and for the Treatment 5 (varenicline), lasting 12 weeks, the interview was applied 3 times, 1 time at the end of each month of treatment; always considering in each interview, the evaluation of the last two weeks before the new interview, according considerations Bech (1993).
17. The physician scored from 0 to 4 points each item, assessing both the intensity and the frequency of it. The total score was obtained from the sum of the scores of each of the items. The range is from 0 to 56 points. The scale does not provide cutoff points to distinguish people with and without anxiety, so the results were interpreted as a quantification of the intensity (Kellner, 1968; Lozano, 1990), proving particularly useful variations after smoke quitting. The first evaluation in the case of 1 to 4 treatments, was performed on day 7 after smoke quitting; and in the case of the treatment 5, the evaluation was the fixed day for smoke quitting. The last assessment (second one for treatments 1 to 4 and third for treatment 5) was performed after the treatment finish, a day after the last dosage, interviewing the patient about the signs and symptoms of the scale during the last two weeks, prior to the interview; the same period that lasted intake treatment in the case of 1 to 4.
18. According to Kellner (1968) two scores were obtained corresponding to psychological anxiety (items 1, 2, 3, 4, 5, 6 and 14) and the somatic anxiety (items 7, 8, 9, 10, 11, 12, and 13), since the effects of treatments can have different degrees of psychic and somatic symptoms (APA, 2000), resulting useful subscale scores, however for the purposes of this document, only the scale was considered for assessing generalized anxiety, asking about symptoms between anxiety attacks. So, under the criteria of Bech (1993) were considered, for guidance: 0 a 5 points "not anxious", 6 to 14 "Anxiety minor" 15 or more "Anxiety greater.".
19. Data were analyzed by statistical software, using ANOVA and statistically significant differences were obtained, then the results are shown:
20. The persons who used the treatment 1, ZAM ® (activated and micronized zeolite) showed lower anxiety before and after treatment, even they stopped smoking; and reported less anxiety than they had the last time they tried to quit, both persons tried to quit smoking before with other kind of treatments. Tracking by phone and two weeks after completing the treatment 1, both persons remained without smoking.
21. The persons who used the Treatment 2, Raspberry Powder (registered patent application number Mx/a/2010/000827, file, folio MX/E/2010/004489) showed lower anxiety before treatment and not anxiety at the end of this. Both persons stopped smoking during the proposed period of treatment (21 days). Tracking was made by telephone every month, and even four months after the end of treatment, both persons still were able to be without smoking; one of them during the third month felt the desire of smoking, but was able to stay without smoking.
22. The person who used the treatment 3, a commercial product of raspberry, similar to the raspberry powder mentioned in the previous section in combination with ZAM ® (activated and micronized zeolite) showed higher Anxiety before the treatment and minor Anxiety features at the end. The person quit smoking in the proposed period (21 days). Monitoring was conducted by telephone each month, and a month after the treatment, went back to smoke again; then this person asked to receive treatment again, which was provided to him and relapsed after a week, four months after the end of treatment, the person was still smoking.
23. In the case of the persons who used the treatment 4, raspberry powder (registered patent application number MX/a/2010/000827 file, folio MX/E/2010/004489) in combination with ZAM ® (micronized and activated zeolite), one of them had higher anxiety before treatment and the other less anxiety. After treatment the first had lower anxiety and second No anxiety. followed by telephone every month and four months after the end of treatment, both were still without smoking.
24. The person who used the treatment 5, varenicline showed less anxiety during the first month of treatment and increased anxiety during the second and third month. Monitoring by telephone every month was conducted, and the person reported that quit smoking in the second month of treatment, remaining smoke-free for two months; but also reported increased anxiety caused to fall smoking again, one month after treatment ends.
25. Fagerström test (Table 2) allows the measurement of the degree of physical dependence that smokers have for the nicotine, is one of the most significant findings in the clinical examination of smoking (Peto, 1996). Fagerström test has proven to be the most useful tool among those available to measure such dependence (Salleras, 1994). It has been the most universally used and with better quality parameters (Plans, 1995). This is a test with six items with multiple answers. Depending on the answer that each one of smoker gives to each of the questions you get a certain score. By sum the points earned in each of the issues a total score between 0 and 10 points is obtained.

**Table 2**

| Question | | Answer | score |
|---|---|---|---|
| How much time passes between waking up and smoke your first cigarette? | | < 5 minutes | 3 |
| | | 6-30 minutes | 2 |
| | | 31-61 minutes | 1 |
| | | After One hour | 0 |
| | | | |
| Find hard to stop smoking in places where it is forbidden to do what? | | Yes | 1 |
| | | No | 0 |
| | | | |
| Of all cigarettes consumed in the day, which needs more? | | The first in the morning | 1 |
| | | Any other | 0 |
| | | | |
| How many cigarettes do you smoke a day? | | 31 or more | 3 |
| | | 21-30 | 2 |
| | | 11-20 | 1 |
| | | 10 or less | 0 |
| | | | |
| After consuming the first cigarette of the day, smoke some more quickly? | | Yes | 1 |
| | | No | 0 |
| | | | |
| Do you smoke as though this ill need to stay in bed most of the day? | | Yes | 1 |
| | | No | 0 |
| | | TOTAL | |
| 0-1 | Very low physical dependence. | | |
| 2-3 | Low physical dependence. From this point it is convenient to use pharmacological treatment to achieve cessation of smoking. | | |
| 4-5 | Moderate physical dependence. A significant risk associated with consumption of snuff disease appears. | | |
| 6-7 | High physical dependence. | | |
| 8-10 | Extreme degree of physical dependence. Necessary to use drug therapy in parallel to a psychological treatment. | | |

26. The assessment of the test, not only serves to ascertain the degree of physical dependence that smoking has by the nicotine, but also can be used for prognostic purposes and therapeutic indication (Jiménez, 2000). The evaluation is done according to the following scheme: From 0 to 1 points the degree of physical dependence is very low. From 2 to 3 points shows a low degree of dependence. The use of pharmacotherapy for smoking cessation in this group of patients is helpful. Of 4-5 points indicates moderate degree of physical dependence on nicotine and a significant risk of illness associated with the consumption of tobacco. Approximately 30% of smokers have this score and in their attempts to quit tobacco consumption should use pharmacotherapy (Jiménez, 2000). Smokers with 6 or 7 points suffer high physical dependence and are at high risk associated with the consumption of tobacco diseases. 15% of smokers get this score. According to Jiménez (2000) is essential to use drug therapy to quit smoking when making a serious attempt to abandonment. From 8-10 points indicate extreme degree of dependence. 5% of smokers get this score and your risk of developing associated diseases to tobacco consumption is very high.
27. In a research study (Prieto, 2003) concluded that 90% of smokers smoked daily and only 1, 7% did so weekly, finding that the average consumption was 15.21 cigarettes / day. 44% of smokers had nicotine dependence (assessed with a score equal to or greater than 5 on the Fagerström test), although the dependence was variable. The main motivation for continuing smoking was the pleasure (30.5%), followed by the routine habits (27.1 %) and the feeling of relaxation (15.3%).
28. Under these considerations Fagerström test and treatment was given, as described above, to the 8 patients referenced herein. The following results were obtained:
29. Of those who used the treatment 1 (activated and micronized zeolite), one of them presented low physical dependence and other moderate physical dependence before treatment, both, as described before, quit smoking and after
30. The persons who used the Treatment 2, (Raspberry Powder (registered patent application number Mx/a/2010/000827, folio MX/E/2010/004489) showed moderate physical dependence before treatment, and finished showing very low physical dependence. Tracking was made by telephone every month, and four months after the end of treatment, they still maintained without smoking.
31. The person who used the treatment 3 (a commercial product of raspberry, similar to the raspberry powder mentioned in the previous section in combination with ZAM ® (activated and micronized zeolite) presented moderate physical dependence before treatment and low physical dependence at the end. However, tracking was made by telephone each month, and four months after the person started to smoke again.
32. Of the persons who used the treatment 4, (raspberry powder (registered patent application number Mx/a/2010/000827 file, folio MX/E/2010/004489) in combination with ZAM ® (micronized and activated zeolite), one of them presented moderate physical dependence before treatment and the other low physical dependence. After treatment both showed very low physical dependence. Tracking was made by telephone every month, and four months after the end of treatment, they still were comfortable without smoking.
33. The person who used the treatment 5, (varenicline) presented moderate physical dependence before treatment, and high at the end of it. Tracking was made by telephone every month and reported having stopped smoking for 2 months, but then have fall smoking in the third month, and the person continue smoking.
34. The attention protocol of patients with treatments 1 to 4 was crucial for best results, so that patent applicants, we are exposing to also protect this application. which is described below:
**Patient care protocol.**

35. On a first meet to patients is asked to them to list the reasons why you smoke and why you have to leave; on that same meet, using a cognitive-behavioral process, the hazards and risks are explained that the snuff smoke implies for the health of himself and those around him; He need to set a date to quit smoking and is given a list of circumstances that allow you to measure the chances of success and they are required to analyze them and fill out the questionnaire containing this list before going to the next appointment.
36. In a second date is discussed with the patient the date to quitting and is reinforced on the fact that should be a significant date for him, while analyzes and reinforces the list of circumstances that speak of the possibilities successful smoking cessation. Selected the date is programmed a third date, it will happen five days before the date fixed for smoking cessation program. A list of activities to be solved by the patient before going to the third date come is given, prompts make a diary in which you must record each cigarette smoked and why it does; are asked to think, before lighting each cigarette, because it does; is asked to whether it is really necessary, and if you considered this you can smoke. You are asked to delay a little every day the first cigarette of the morning. You are instructed not to accumulate snuff, advising buy one package at a time. You are invited to try smoking in a spaced form and not smoke the cigarettes that would not be entirely necessary and to turn them off in the middle. You are instructed to refuse offers of snuff other smokers and think of the fact that although fall into any of the deals, gradually get used to reject them. It makes you reflect on snuff trap "light", indicating that the fact of having less nicotine can lead to smoke more often and take deeper puffs. You are instructed to practice any physical activity you can do regularly (walking, running, cycling, etc.). You are invited to comment to the people around you that you will quit in a few days. You are encouraged to seek company and to form a group to know that you can support to overcome the bad times together. It makes you think you can ask family and / or friends who are part of the support group, who can go, even by telephone, to overcome some point of crisis, the physician and / or therapist phone are provides the or treating institution.
37. From that moment appointment is given 5 days prior to the date fixed for quitting. On the third appointment, is forewarned that from this moment you have 5 days to quit and reviewed with the patient what they should do every day:
38. First Day, should set the time to quit. You are invited to talk again with your friends and family of your plan. You are prompted to stop buying cigarettes and write the date in a "Letter of Commitment", which specify the time, day, month and year will be to quit.
39. Second Day. You are prompted to make another list of when and why you smoke. It invites you to think of that day in new ways to relax, things can get his hands instead of a cigarette, you are invited to analyze habits or routines that you want to change and are requested write this into a List.
40. Third Day. will be show on that day make a list of things you can do with the money you'll save by quitting. You are invited to call a friend, an ex-smoker or your support group when you need help.
41. Fourth Day. You are told to take 1 capsule of 500 mg of composed Valerian each / 8 hours. For 5 days. You are instructed to wash their clothing, and bedding to remove cigarette smell.
42. Fifth Day is suggested that you choose a reward and the acquiring, to give it to yourself after you quit. You are instructed to make an appointment with your doctor and / or treating institution. It tells you that the end of the day all cigarettes and matches be discarded, and keep lighters and ashtrays
43. Final Day. This day is the date set in the first day. It tells you kept well occupied, you change your routine when possible and do things out of the usual. It tells you to remind your family, friends and work colleagues that this is the day to quit and invites you to ask them to give you help and support.
44. Day After. Congratulate yourself and your reward be purchased, a gift or do something to celebrate. You are instructed to avoid alcohol. It is suggested that when you want a cigarette, you do something that is not related to smoking, such as a walk in the park, take a glass of water, or deep breathing.
45. Upon completion of the third appointment are delivered in writing the above indications and gather to the day after the date for quitting. Are also given, in writing, information on what to do in case of anxiety, constipation, hunger and/or weakness.
46. The fourth appointment is scheduled for the 6th day, after starting the countdown 5 days to the date specified has quit. At this appointment the patient is advised about treatments that support the process of quitting, advantages and disadvantages are communicated. Are explain what the process of detoxification of the body and is guided on using the treatment with the product, resulting from the combination of the ZAM ® (micronized and activated zeolite), as described in the trademark registration, and raspberry powder (according to process registered in the Mexican Institute of Industrial Property, in patent application number Mx/a/2010/000827 record dated 21/ENE/2010, folio MX/E/2010 / 004,489), subject to registration of this patent document. Treatment with this product is indicated for 14 days at doses of 7 g of powder diluted, preferably in fruit juice or any other drink except alcohol and suggesting that ingested daily at the same time, preferably in the morning.
47. The base powder reconstituted with water retains its content of minerals such as calcium, magnesium, potassium, silicon, sodium, manganese, sulfates and phosphates, carbohydrates and nitrogenous compounds, as well as vitamins A, B1, B2 and C, the latter will give the quality of being an excellent antioxidant to counter free radicals, being vitamins preferred, but it is feasible and it is permissible to add any other vitamin supplement. Likewise retains chemical components ZAM ® (activated and micronized zeolite) SiO2, CaO, K2O, MgO, Na2O by strengthening antioxidant and detoxificant effect by be mineral negatively charged microcrystalline and naturally attract and adsorb positively charged toxicants because either air or the skin and blood. The product covered by this application can be used for the treatment of anxiety caused by nicotine withdrawal, and to control the need of nicotine, because it detoxify the body of this substance and others present in the tobacco smoke. It can be used diluted in water or in any permissible drink except alcohol, or as an additive in any food such as cookies, jellies, cakes, gelatins, yogurt, among others, as well as other personal care products such as oral rinses, toothpaste, and skin creams, among others cleaning products like laundry detergents, soap for the whole body, shampoo, among others, cigarette filters and others.
48. The base powder has a shelf life of more than one year while retaining its original properties.

Having sufficiently described the invention, it is considered as a new product, process and therapeutic properties obtained from it and therefore claim as our exclusive property contained in the following claims.

## Claims

1. Process for obtaining a base powder juice made from red raspberry Rubus ideaus and optionally dehydrated fruit from the same plant, with appropriate ripeness and appropriate sanitation and ZAM ® (activated and micronized zeolite) to prepare functional drinks, refreshing drinks or food additive, for food products, such process is **characterized by** the next steps:
a) use fresh raspberry;
b) is cleaned by removing the peduncles, pedicels and leaves;
c) raspberry juice extract is obtained using for example any of the following methods:
i. Marmite method - A marmite with fresh fruit crushed previously, heated slowly without exceeding 70 to 80 ° C, so the fruitjuice begins to flow, during approximately 20 minutes per kg of fruit;
ii. Bath method - Crushed fresh fruit is placed in a container and placed in a water bath until it begins to flow fruit juice, not exceeding a temperature of 70 ° C to 80 ° C;
d) once raspberry juice was obtained, it is standardized with a thickener agent and zeolite is added;
e) drying the juice standardized.

2. Process according to claim 1, **characterized by** after step (e), optionally, a sweetener can be added.

3. Process according to claim 2, **characterized by** the sweetener may be sugar.

4. Process according to claim 2, **characterized in that** the sweetener can be fructose.

5. Process according to claim 2, **characterized in that** the sweetener can be aspartame.

6. Process according to claim 1, **characterized in that** after step (e), and optionally according to claim 2, after addition of a sweetener, anti-humectants may be added to the product obtained by drying the juice

7. Process according to claim 6, **characterized by** that to the formulations indicated in claims 1 to 5, any anti- humectant is added to them, such as silicon dioxide or tricalcium phosphate or any others authorized by the corresponding rules.

8. Process for obtaining a base powder juice made from red raspberry Rubus ideaus juice and optionally from dehydrated fruit from the same plant, with appropriate ripeness and appropriate sanity conditions and with ZAM ® (activated and micronized zeolite) to prepare functional drinks, refreshing drinks or as food additive according to claim 1, and optionally according to claims 2 to 7, **characterized by** a filtration process .

9. Process according to claim 8, **characterized in that** the filtering is performed using filter paper of average pore.

10. Process according to claim 1, **characterized by** that in an optional step (d), raspberry juice is standardized with a thickening agent and zeolite is added.

11. Process according to claim 10, **characterized by** the thickening agent is any thickening and stabilizer agent, authorized by the corresponding legislation, preferably maltodextrin and gum Arabic, or xanthan gum besides others and the zeolite is a zeolite-Clinoptilolite known as ZAM ® (activated and micronized zeolite).

12. Process according to claim 1, **characterized by** it includes a drying step.

13. Process according to claim 12, **characterized in that**, from the standardized raspberry juice extract, a powder is obtained by a spray drying process.

14. Process according to claim 13, **characterized in that** the drying is done using a spray-dryer device type.

15. Drying process according to claim 12, **characterized in that** the drying is by lyophilization

16. Process according to any of the preceding claims, **characterized by** that the ingredients of any of the formulations described in claims 1 to 15 are mixed and homogenized to obtain the final base powder.

17. Base powder to preparation functional beverages, refreshing drinks, or as an additive in food preparations, **characterized by** it is formulated with dehydrated fruit juice Red Raspberry from Rubus ideaus and ZAM ® (active and micronized zeolite), obtained according to the Claims 1 to 16.

18. Homogenate product obtained from the process of claims 1 to 17, **characterized by** that it can be stored in glass jar with lid twist off and plastic seal, or in pouch of laminated polyethylene-aluminum and glassed paper, or any other method of preservation allowed by the corresponding legislation.

19. Product according to claim 18, **characterized in that** it has a shelf life of more than 1 year according to reference in the description.

20. Process according to claim 1, letter (c), **characterized by** the raspberry juice extract, is obtained using a marmite with the fresh fruit previously crushed and heating slowly without exceeding 70 to 80 ° C, in order to favor the preservation ellagic acid above other products.

21. Process according to claim 1, letter (d), **characterized by** the raspberry juice extract, is standardized to a concentration of 20% to 35% solids with maltodextrin arabic gum or nopal mucilage and ZAM ® (activated and micronized zeolite) in a ratio of 64.94% raspberry juice, 25.96% maltodextrin, 3.90% arabic gum or nopal mucilage and 5.20% of ZAM ® (activated and micronized zeolite).

22. Process according to claim 1, letter (e), **characterized in that** the extract standardized raspberry juice, is subjected to spray drying process using a spray-dryer equipment to an inlet temperature of 110 ° C and outlet of 80 ° C, with an air flow of 600 ml / min and vacuum 30 millibars.

23. Base powder for functional beverages preparation, refreshing drinks, or as an additive in food preparations, formulated with pulp and dehydrated raspberry juice from fruit of Rubus ideaus red and ZAM ® (micronized and activated zeolite), according to the process described in the claims 1 to 5, **characterized by** sugar, fructose or aspartame is used, as any other or sweetener.

24. Base powder for functional beverages preparation, refreshing drinks, or as an additive in food preparations, **characterized in that** silicon dioxide or tricalcium phosphate is used as anti-humectant agent according to claims 1 to 7.

25. Base powder for preparation of functional beverages, refreshing drinks or as an additive in food preparations, **characterized by** the use of maltodextrin and gum arabic or nopal mucilage and gum arabic besides ZAM ® (micronized and activated zeolite) among others thickeners agents and stabilizers according to the process described in claims 1 to 1 1.

26. Base powder for preparation functional beverages, refreshing drinks, or as an additive in food preparations, **characterized in that** the powder has a shelf life of over one year shelf, according to the process described in claims 1 to 19.

27. Base powder for preparation functional beverages, refreshing drinks or as an additive in food preparations, **characterized by** the product is rehydrated with water, milk, fruit juice, or other ingestible liquid, instantly providing a acid-sweet sui generis flavor solution according to claims 1 to 19.

28. Base powder for preparation functional beverages, refreshing drinks or as an additive in food preparations, **characterized by** the product is rehydrated with water, milk, fruit juice, or other ingestible liquid, except alcoholic beverages, instantly and retains all the properties of the fruit and the ZAM ® (micronized and activated zeolite), the color and flavor may be changed if water is not used, according to claims 1 to 19.

29. Base powder for preparation functional beverages, refreshing drinks, or as an additive in food preparations, according to claim 27, **characterized by** the product is rehydrated with water, milk, fruit juice, or other ingestible liquid, except alcoholic drinks, instantly and retains all its minerals like calcium, iron, aluminum, magnesium, potassium, silica, sodium, manganese, sulfates and phosphates, carbohydrates and nitrogenous compounds, plus vitamins A, B1, B2 and C and the ellagic acid, whose conservation is intentionally favored by the same process of obtaining and preserves chemical components of the ZAM ® (micronized and activated zeolite) SiO2, CaO, K2O, MgO, Na2O, potentiating the antioxidant and detoxifying effect by the negatively charged microcrystalline mineral and naturally attract and adsorb positively charged toxic substances, either from air or the skin and blood. As a toxin adsorbent as NH3, hydrocarbons, C1-C4, CO2, H2S, SO2, NOx, aldehydes, in addition to heavy metals like lead, cadmium, cesium, mercury, etc., and listed in the "Generally Recognized As Safe (GRAS)" and the U.S. Code of Federal Regulations (21 CFR 182, Subpart C).

30. Base powder for preparation functional beverages, refreshing drinks, or as an additive in food preparations, according to claim 27, **characterized in that** the product is rehydrated with water, milk, fruit juice, or other ingestible liquid, except alcoholic beverages, in instant form and where, according to claim 29 vitamins A, B1, B2 and C, ellagic acid itself and the chemical components of the ZAM ® (activated and micronized zeolite) give it the quality of being an antioxidant to counteract free radicals, plus raspberry powder can be added with any vitamin supplement.

31. Base powder for preparation of functional beverages, refreshing drinks, or as an additive in food preparations, according to claim 27, **characterized by** the product is rehydrated with water, milk, fruit juice, or other ingestible liquid, except alcoholic beverages, instantly, according to claims 29 and 30, retains all the therapeutic properties, among them we can include: antioxidant, antimutagenic, anticancer and immunostimulant and detoxification by the use of nicotine and other toxic substances in the different presentations of tobacco and as an anxiolytic for its content of ellagic acid content and ZAM ® (activated and micronized zeolite).

32. Base powder for preparation functional beverages, refreshing drinks, or as an additive in food preparations, according to claim 27, **characterized by** the product is rehydrated with water, milk, fruit juice, or other ingestible liquid, except alcoholic beverages, in instant form and where, according to claim 31, their contents of ellagic acid and ZAM ® (activated and micronized zeolite), give it the quality of being a nicotine detoxifying and other toxic substances detoxifying present in tobacco smoke, and in addition helps in the control of anxiety caused by withdrawal syndrome when nicotine is suppressed and helps in controlling nicotine addiction eradicating the need for further doses of nicotine, once the addiction is established, so it can be recommended as an aid in the control of active and passive tobacco smoking, which makes it a support control of nicotine addiction and encourages a process of detoxification in smokers and nonsmokers exposed the tobacco smoke.

33. Base powder for preparation functional beverages, refreshing drinks, or as an additive in food preparations, according to claim 27, **characterized by** the product is rehydrated with water, milk, fruit juice, or other ingestible liquid, except alcoholic beverages, instantaneously and where, according to claim 31, the content of ellagic acid confers the product the property of being an anxiolytic, by controlling the anxiety independently of the administration or suspension of nicotine. Also, the content of ZAM ® (micronized and activated zeolite), according to claim 31, enhances the antioxidant and detoxifying effect by being micro-crystalline negatively charged minerals and naturally attract and adsorb toxics substances positively charged, whether from air or from the skin and the blood, favoring also an immunostimulating effect.

34. Basis powdered for preparation of functional beverages, refreshing drinks, or as an additive in food preparations, according to claim 27, **characterized by** the product is rehydrated with water, milk, fruit juice, or other ingestible liquid, except alcoholic beverages, instantly, and where according to claim 29, their content of ellagic acid is favored by the same obtaining process and a concentration of free ellagic acid is of 1.5 g/kg of powder (1500 ppm), and hydrolyzed (total ellagic acid) of 3.2 g / kg of powder (3200 ppm).

35. Basis Powder for preparation of functional beverages, refreshing drinks, or as an additive in food preparations, according to claim 27, **characterized in that** the product is rehydrated with water, milk, fruit juice, or other ingestible liquid, except alcoholic beverages, instantly and where, according to claim 34 the suggested human dose can range from 5 g to 10 g per day to get the benefits, without necessarily being those doses a limit, because the use may vary according to the characteristics of each individual and by tobacco smoke exposure and number of cigarettes or tobacco amount in any of its presentations.
